(19) Europäisches Patentamt
European Patent Office
Office européen des brevets

(11) Publication number: **0 381 527 A1**

(12) # EUROPEAN PATENT APPLICATION

(21) Application number: 90301136.9

(22) Date of filing: 02.02.90

(51) Int. Cl.5: C07C 59/42, C07C 59/125, C07C 69/708, A61K 31/19, A61K 31/22

(30) Priority: 02.02.89 IL 89155

(43) Date of publication of application: 08.08.90 Bulletin 90/32

(84) Designated Contracting States: AT BE CH DE DK ES FR GB GR IT LI LU NL SE

(71) Applicant: YISSUM RESEARCH DEVELOPMENT COMPANY OF THE HEBREW UNIVERSITY OF JERUSALEM
46 Jabotinsky Street
Jerusalem, 92 182(IL)

(72) Inventor: Lichtstein, David
22 Propes Street
Ramot 97725(IL)
Inventor: Deutsch, Joseph
21 Hameshoreret Rachel Street
Jerusalem(IL)

(74) Representative: Collier, Jeremy Austin Grey et al
J.A.Kemp & Co. 14, South Square Gray's Inn
London WC1R 5EU(GB)

(54) **Ouabain like compounds and pharmaceutical compositions containing them.**

(57) Compounds having digitalis-like activity are isolated from dried mammalian plasma. They may have the formula:

$R_1-CH=CH-CH(OH)-CH_2-CH(OH)-(CH_2)_n-COOH$ (I)

wherein $R_1$ represents a $C_{1-6}$ straight or branched alkyl radical and n is an integer of from 2 to 11, and pharmaceutically acceptable salts thereof.
or;

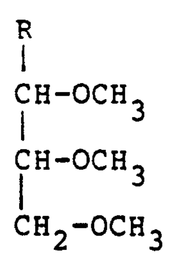

(II)

wherein R represents an alkylenecarboxy, alkylenecarbonyloxy-alkyl, carbonyloxy-alkyl or alkyleneoxy-alkylene-carbonyloxyalkyl radical.

EP 0 381 527 A1

## OUABAIN-LIKE COMPOUNDS AND PHARMACEUTICAL COMPOSITIONS CONTAINING THEM

The digitalis group compounds such as digoxin, digitoxin or ouabain are extracted from the dried leaves of the common purple foxglove, digitalis purpurea, and the seeds and leaves of some other plants. Such extracts have been used for treatment of chronic heart failure for over two hundred years. In general, the digitalis group compounds, often referred to as cardiac glycosides, are powerful cardiotonic agents, increasing the force of muscle contractions and slowing the rate of the heart beat under certain conditions.

The molecular mechanism by which digitalis compound exert their pharmacological effects is their ability to inhibit activity of the Na,K-ATPase (E.C.3.6.1.3). The Na,K-ATPase is a transmembranal enzyme which hydrolyses ATP and the free energy released by this hydrolysis is used by the same enzyme to transport $Na^+$ out of and $K^+$ into the cells. Digitalis binds specifically to this enzyme and inhibits its activity (for review on Na,K-ATPase and the effect of digitalis on this enzyme see Glynn, I.M. et al., J. Ann. Rev. Physiol. 37: 13-55 (1975); Lindenmeyer, G.A., Pharmacol. Ther. 2: 843-861 (1976)). Compounds which are structurally related to the digitalis group compounds have, until some years ago, been only shown to exist in the poison glands of bufonids toads and in the skin of several species of amphibia. Still, the existence in animals and humans of endogenous digitalis-like compounds (hereafter DLC) was seriously contemplated. Such endogenous digitalis-like compounds were postulated to be the natural ligands for the digitalis receptor on the Na,K-ATPase and regulators of the activity of this plasma membrane enzyme. Indeed, in recent years evidence has been accumulated to the existence of "digitals-like" (or "ouabain-like") compounds, in extracts from mammalian brain (Haupert G. T., Jr., et al., Proc. Natl.. Acad. Sci. U.S.A. 76: 4658-4661 (1979); Fishman M.C., Proc. Natl. Acad. Sci. U.S.A. 76: 4661-4663 (1979); Lichtstein D., et al., Biochem. Biophys. Res. Commun. 96: 1518-1523 (1980)), heart (De Pover A., et al., Biochem. Pharmacol. 31: 267-271 (1982)), adrenal (Tamura M., et al., Biochemistry 27: 4244-4253 (1988)), plasma (Haddy F.J., et al., Life Sci. 19: 935-948 (1976); Hamlyn J.M., et al., Hypertension 10: I-71-177 (1987)) and urine (Goto A., et al., Biochem. Biophys. Res. Commun. 154: 857-853 (1988); Cloix J.F., et al., Can. J. Physiol. Pharmacol. 65: 1522-1527 (1987)) which exhibit a ouabain-like activity. Increasing evidence demonstrates that the levels of these compounds are increased in extracellular volume expansion of animals, suggesting their role in volume and salt homeostasis (for review see Haber E., et al., Hypertension 9: 315-324 (1987)).

Since digitalis group compounds inhibit Na,K-ATPase, they affect numerous systems in which this enzyme is involved. Thus, these drugs are potent cardiotonic agents known for their positive ionotropic action, as well as affecting the heart beat rate under certain conditions. In addition, cardiac glycosides affect the excretion of $Na^+$ ions in the kindney, causing natriuresis and, at higher concentrations, have several effects on the central nervous system (for review see Hofmann B.E., et al., in the Pharmacological Basis of Therapeutics (Goodman and Gilman Eds.), Macmillan Publishing Co. Inc. N.Y. (1980)).

Several substances have been proposed as the DLC, including unsaturated fatty acids (Kelly R.A., et al., J. Biol. Chem. 260: 11396-11405 (1985)) and lysophospholipids (Hamlyn J.M., et al., Hypertension 10: I-71-I77 (1987)). However, none of these compounds appears to be the natural ligand of the receptor of the Na,K-ATPase because of their limited specifity and affinity. A specific endogenous inhibitor of Na,K-ATPase has been recently purified from bovine adrenal (Tamura M., et al., Biochemistry 27: 4244-4253 (1988)). This inhibitor is described as a small water soluble organic compound, having a molecular weight of 336. However, the structure of this compound is still to be elucidated. Present inventors have recently shown (Lichtstein D., et al., Life Sci. 38: 1261-1270 (1986)) the presence in the toad skin and plasma of a bufodienolide derivative, which answers the above criteria, however its physiological role in the toad, or its presence in mammals, have yet to be investigated. All of the unidentified endogenous compounds described heretofore had the common feature of interacting with the ouabain binding site of the Na,K-ATPase and inhibiting its activity, and, like digitalis, increase the force of contraction of the heart muscle (Shimoni Y., et al., Nature. 307: 369-371 (1984)). It was generally agreed that the DLC is/are of low molecular weight (<1,000d), soluble in water and methanol but insoluble in chloroform or hexane.

Availability of endogenous digitalis-like compounds and the possibility of their administration as therapeutic agents may result in advantageous treatment of the above described pathological conditions.

## DESCRIPTION OF THE INVENTION

In search for endogenous digitalis-like compounds, some such compounds have now been purified to homogenity from bovine plasma and their structure identified.

The invention therefore relates to compounds of the general formula:

$$R_1\text{-}CH=CH\text{-}\underset{OH}{CH}\text{-}CH_2\text{-}\underset{OH}{CH}\text{-}(CH_2)_n\text{-}COOH \qquad (I)$$

wherein $R_1$ represents a $C_{1-6}$ straight or branched alkyl radical and n is an integer of from 2 to 11 and pharmaceutically acceptable salts thereof.

Preferred compounds are compounds of formula I wherein $R_1$ represents a $C_{3-6}$ alkyl radical.

Most preferred compound of formula I is the compound wherein $R_1$ represents propyl and n is 9, namely 11,13-dihydroxy-14-octadecaenoic acid as well as pharmaceutically acceptable salts thereof.

The isolation and characterization of compounds of formula I are described hereafter in the Examples. The digitalis-like activity of these compounds is also described in the Examples.

In a further embodiment, the invention relates to compounds of the general formula:

$$\begin{array}{l} R \\ | \\ CH\text{-}OCH_3 \\ | \\ CH\text{-}OCH_3 \\ | \\ CH_2\text{-}OCH_3 \end{array} \qquad (II)$$

wherein R represents an alkylenecarboxy, an alkylenecarbonyloxyalkyl, a carbonyloxyalkyl or an alkyleneoxyalkylenecarbonyloxyalkyl radical.

Preferred compounds according to formula II are compounds in which R represents the radicals $-CH_2-(CH_2)_{12}COOH$, $-CH_2-OCO(CH_2)_{11}CH_3$, $-OCO(CH_2)_{12}CH_3$ or

$$-CH_2\text{-}0\text{-}(CH_2)_{12}\text{-}\overset{O}{\overset{\|}{C}}\,OC_2H_5$$

The isolation and characterization of compounds of formula II are described hereafter in the Examples. The digitalis-like activity of these compounds is also described in the Examples.

According to a feature of the invention, products having digitalis-like activity are isolated by a process which comprises subjecting dried mammalian plasma to extraction with methanol, washing the methanolic extract with a volatile liquid aliphatic hydrocarbon, evaporating the washed methanolic extract to dryness, subjecting the dried extract to chromatographic separation, and isolating fractions showing digitalis-like activity as determined by bioassay.

The invention is also concerned with pharmaceutical preparations for the treatment or prevention of cardiac malfunction or renal malfunction comprising as active ingredient a compound of formula I or a pharmaceutically acceptable salt thereof, optionally further comprising pharmaceutically acceptable additives in a suitable carrier or diluent.

Preferred preparations comprise as active ingredient 11,13-dihydroxy-14-octadecaenoic acid or pharmaceutically acceptable salts thereof.

In another embodiment the invention is concerned with pharmaceutical preparations for the treatment or prevention of cardiac malfunction or renal malfunction comprising as active ingredient a compound of formula II, optionally further comprising pharmaceutically acceptable additives in a suitable carrier or diluent. Preparation comprising as active ingredients suitable mixtures of at least two compounds of formula II are also envisaged. Preferred preparations comprise as active ingredients compounds of formula II wherein R represents the radical

$-CH_2(CH_2)_{12}COOH$ ,$-CH_2-OCO(CH_2)_{11}CH_3$, $-OCO(CH_2)_{12}CH_3$ or

$$-CH_2\text{-}0\text{-}(CH_2)_{12}\ \overset{O}{\overset{\|}{C}}\,OC_2H_5.$$

Also within the scope of the present invention are pharmaceutical preparations comprising as active ingredient suitable mixtures of a compound of formula I with at least one compound of formula II, optionally further comprising pharmaceutically acceptable additives in a suitable carrier or diluent.

The preparations of the present invention may be used for the treatment of malfunctions such as cardiac failure and arrhythmia and for the induction of natriuresis under pathological conditions.

The preparations according to the invention may be administered orally or parenterally in dosage unit formulations containing conventional nontoxic pharmaceutically acceptable carriers, diluents, adjuvants and vehicles as desired. The term parenteral as used herein includes subcutaneous, intravenous, intramuscular, intraarticular and intrathecal injection and infusion techniques.

Dosage unit preparations may contain daily required amounts of the compounds of the invention or submultiples thereof to make up the desired dose. The specific therapeutically effective dose level for any

particular patient will depend upon a variety of factors including the activity of the specific compound employed, the age, body weight, general health, sex, diet, time of administration, route of administration, rate of excretion etc.

As used herein, the term "pharmaceutically acceptable carrier" means an inert, non-toxic solid or liquid filler, diluent or encapsulating material, not reacting with the active ingredients of the invention. These carriers are known to the man versed in the art. Wetting agents, emulsifiers and lubricants, as well as coloring agents, release agents, coating agents, sweetening and flavoring agents and preservatives can also be present in the preparations of the invention. The amount of active ingredient that may be combined with the carrier materials to produce a single dosage form will vary depending upon the patient treated and the particular mode of administration.

## BRIEF DESCRIPTION OF THE FIGURES

Figure 1: Chromatography of 67% acetonitrile fraction of methanolic extract of bovine plasma (see Example 1).

Figure 2: Chromatography of fractions obtained by the chromatography described in Fig. 1.

Figure 3: Separation of Peak II of Fig. 2 on analytical reverse phase column.

Figure 4: Separation of Peak III of Fig. 2 on analytical reverse phase column.

Figure 5: Mass spectrum of DLC-II.

Figure 6: DLC-II and possible fragments and isomers.

Figure 7: Mass spectrum of DLC-III.

Figure 8: Fragments of DLC-III.

Figure 9: DLC-III - Compounds of Formula II.

Figure 10: Biological activity of DLC-I, DLC-II and DLC-III.

## EXAMPLES

### MATERIALS AND GENERAL METHODS

#### Materials:

[$^3$H]-ouabain was purchased from New England Nuclear. Organic solvents were of high-performance liquid chromotography (HPLC) grade. Other chemicals were of analytical grade.

#### Extraction of DLC from Bovine Plasma:

150 Kg of lyophilized bovine plasma, equivalent to approximately 2000 liters of plasma, were used. DLC were extracted from the plasma by 10 volumes of methanol. Following filtration, the methanolic solution was concentrated and washed with hexane. The methanolic layer was separated and evaporated to dryness and the resulting material applied to a low pressure C-8 column which was developed with increasing concentrations of acetonitrile. The 67% acetonitrile fraction which contained most of the DLC activity as determined by several independent bioassays for DLC activity, described hereafter, was further purified using high performance liquid chromotography [HPLC) as described hereafter in Example 1.

#### Assays for Digitalis-Like Activity:

The digitalis-like activity of each fraction obtained in the course of the purification was determined quantitatively using at least two of the following bioassays:

**a. Inhibition of [$^3$H]-ouabain binding:**

The binding of [$^3$H]-ouabain to rat brain synaptosomal fraction was carried out by a conventional filtration technique as previously described (Lichtstein D., et al., Life Sci. 38: 1261-1270 (1986)). In brief: The reaction mixture (500µl final volume) contained the following constituents (final concentrations): 50 mM Tris-HCl buffer (pH 7.4), 0.5 mM EDTA, 80 mM NaCl, 4 mM $MgSO_4$, 2 mM ATP (Tris, vanadium-free) and 32 nM [$^3$H]-ouabain (19.5 Ci/mmol). Reactions were initiated by the addition of 200 µl of crude synaptosomal fraction resulting in a final protein concentration of 200 µg per reaction. Following incubation for 1hr. at 37°C, the reactions were terminated by the addition of 3 ml cold (4°C) 50 mM Tris-HCl (pH 7.4) and filtered over Whatman GF/B filters. The filters were washed two times with 3 ml volumes of the same Tris buffer, dried and assayed for radioactivity in a Packard liquid scintillation counter at a counting efficiency of approximately 50%. Specific binding was calculated by subtracting the binding observed in thc presence of 100 µM unlabeled ouabain from that observed in the absence of unlabeled ouabain. Specific binding represented 93% of the total binding under control conditions.

**b. Inhibition of Na, K-ATPase activity:**

Na,K-ATPase activity in rat brain microsomal fraction was determined as previously described (Lichtstein D., et al., Life Sci. 38: 1261-1270 (1986)). Enzyme activity was measured by the colorimetric determination of inorganic phosphate after the incubation of microsomes (200 µg protein per reaction) at 37°C in a solution (final volume 500 µl) containing (final concentrations) 34 mM Tris-HCl (pH 7.4), 100 mM NaCl, 20 mM KCl, 4 Mm $MgCl_2$ and 4 mM ATP (Tris, vanadium free). After preincubation for 10 min., the ATP was added to initiate the reaction. Reactions were terminated by the addition of 100 µl of 5% trichloroacetic acid and the precipitate was removed by centrifugation. Na,K-independent ATPase activity was determined by omitting $Na^+$ and $K^+$ from the reaction mixture or by the addition of 1 mM ouabain to the complete assay mixture.

**c. Inhibition of $^{86}$Rb uptake to N1E115 cells:**

Neuroblastoma N1E115 cells were grown in MEM (Modified Eagle's Medium) medium. Cells were removed from their growth flask by raising and lowering the medium with a pipette and centrifuged at 100 x g in a Sorval RC-5 centrifuge for 10 minutes. The pellet was resuspended in MEM. $^{86}$Rb-uptake was initiated by the addition of N1E115 cells to MEM which contained $^{86}$Rb tracer (1 µCi). Uptake was terminated after 1 min. by the addition of 1.5 ml ice cold 125 mM $MgCl_2$ solution followed by 1 min. centrifugation in an Eppendorf centrifuge. The Eppendorf tube was cut and the pellet removed and transferred to a scintilation vial, to which 6 ml of Emulsifier Scintillator 299TM (Packard) were added and radioactivity was determined.

High performance Liquid Chromotography:

Reverse-phase HPLC were run on a semipreparative Partisil ODS-3 column (1.0x50 cm, 10 µm particle size, Whatman) and analytical Lichrospher RP-18 column (0.4x25 cm, 5µm particle size, Merck) using the LKB 2150 HPLC pump connected to LKB 2152 HPLC controller. The columns were developed with a linear gradient of acetonitrile at flow rates of 1 ml/min. for the analytical columns and 3 ml/min. for the semipreparative columns. The elution of the compounds from the columns was monitored at various wavelengths using the Knauer variable wavelength monitor or by the refraction index monitor using the ERMA Inc. instrument.

NMR and Mass Spectra Determinations:

The $^1$H NMR spectra were obtained on WH-300 Bruker spectrometer with tetramethylsilane as an internal standard and methanol-d4. Ultraviolet spectra were run in methanol on Varian Tectron model 635 spectrophotometer. Low resolution mass spectra were recorded by electron impact ionization on LKB 2091 mass spectrometer at 70 eV. The samples were inserted by direct inlet and heated externally to 300°C at the rate of 50° per minute. The high resolution measurements were performed on a Varian CH5 DF mass spectrometer with a resolution of 10,000.

Na$^+$, K$^+$ and Protein Determinations:

Na$^+$ and K$^+$ were determined by atomic absorption using a Perkin-Elmer atomic absorption spectrometer. Protein concentration was determined by the colorimetric method of Lowry.

EXAMPLE 1

**Purification of DLC from bovine plasma.**

In order to purify the plasma DLC, a methanolic extract was prepared from 150 kg lyophilized bovine plasma as described above. The active methanolic fraction was applied to a low pressure C-18 column developed stepwise with acetonitrile. The 67% acetonitrile fraction, which contained most of the biological activity (data not shown) was further frctionated using preparative, semipreparative and analytical HPLC systems.

Aliquots of the active fraction were injected onto a C-8, reverse phase, preparative column equilibrated with 80% acetonitrile and eluted with a linear 0-80% acetonitrile gradient over 60 min. An example of the UV absorbance and the biological activity patterns obtained in this chromatography is shown in Figure 1.

The active fractions, between 25 to 50 min. retention time, obtained from this chromatography were collected, concen trated and applied to a semipreparative column (Partisil ODS-3 column, 10x50 cm, 10 $\mu$m particle size) eluted at identical conditions. The activity from the semipreparative column was resolved into three activity peaks (Figure 2), which were designated I (retention time of 0-10 min.), II (retention time of 28-38 min.) and III (retention time of 47-57 min.). Peak II was eluted along with the strongest UV absorbance while peak III had no absorbance at 254 nm (Figure 2). The distribution of the activities between the three peaks did vary quantitatively in different preparations, but their retention time was very reproduceable. The materials under the three peaks of activity were further chromatographed using analytical reverse phase chromatography, the separation of peak II and III is further described herein.

The separation of peak II on analytical reverse phase column (Lichrospher RP-18, 0.4X25 cm, 5 $\mu$m particle size) eluted with isocratic system of 50% acetonitrile and monitored at a 254 nm and with a refraction index detector is shown in Figure 3. The DLC-II was eluted at 4 min. retention time, overlapping a peak monitored with the refraction index. The compound represented by this peak was subjected to the mass spectral and NMR analyses (see below).

Figure 4 represents the elution profile of peak III injected onto an analytical reverse phase column (Lichrospher RP-18, 0.4x25 cm, 5 $\mu$m particle size), eluted with isocratic system of 75% acetonitrile. The DLC activity was eluted at 10 min. retention time and did not have any significant UV absorbance either at 254 or 280 nm. However, the refraction index monitor revealed a strong peak which totally superimposed the biological activity (Figure 4, lower panel). This material was subjected to the mass spectral analysis.

EXAMPLE 2

Structures identification

(1) The DLC compound represented by peak II was analyzed by EI mass spectrometer for structure identification. The mass spectrum of this compound is shown in Figure 5. It shows a base peak at 99 atomic units (a.u.) (100%), a small molecular peak at 296 and a larger peak at 278 (35%) due to elimination of a water molecule (18 atomic units). Additional peaks at 171 and 153 were also detected in the spectrum. High resolution measurements of the peak at 278 were performed at a resolution of 10000 using a CH5-DF instrument. The accurate mass found 278.222 a.u., corresponding to the composition $C_{18}H_{30}O_2$ (calculated mass 278.224). Methyl esterification of this compound with diazomethane ethereal solution shifted the peaks 278 and 296 by 14 a.u. proving that a methyl ester of a hydroxy acid is involved in the structure. The esterification process did not shift the 99 and 153 peaks but shifted the 171 peak to 185 a.u.. Taken together, these data suggested that the material has a hydroxyoctadecadienoic acid structure resembling a precursor of linolenic acid (9,12,15-octadecatrienoic acid, MW 278, Figure 6a). The fragment represented by the 99 peak is formed by an alpha cleavage to a hydroxyl group, indicating that a hydroxyl group is attached to $C_{13}$. The presence of peaks at 171 and 153 a.u. and the fact that only the 171 peak was shifted

to 185 by methyl esterification, suggest that the double bond should be located between $C_{11}$ and $C_{12}$ and not as in the 13-hydroxy linolenic acid (Figure 6). Furthermore, considering that the vinyl bonds are not easily cleaved under electron impact, it is reasonable to assume that the 171 fragment is formed by a heterolytic cleavage (alpha to a second hydroxyl group located on $C_{11}$) as shown in Figure 6.

In addition, the high abundance of the 99 peak can be better explained by the location of the double bond at $C_{14}$ posi tion, which enables the formation of stabilized conjugated double bonds (Figure 6). Thus, according to the fragmentation pattern the structure of the DLC present in peak II is suggested to be 11,13-dihydroxy-14-octadecaenoic acid (DLC-II).

DLC-II, being a hydroxylic acid compound, tends to loose one or two molecules of water, losing a large part of its biological activity (see below) which is accompanied by simultaneous disppearance of the 99, 153 and 171 peaks at the mass spectral measurements. The resulting compound is the conjugated hydroxy compound 11-hydroxy-12,14-octadecadienoic acid (Fig. 6(e), not illustrated) represented by the peak at m/z 296 (Fig. 5). Possibly this compound is one of the isomers 13-hydroxy 10,14-octadecadienoic acid or 13-hydroxy-11,14 octadecadienoic acid. These isomers can undergo dehydration and the resulting compound is the hyperconjugated octadeca-11,13,15-trienoic acid (Fig. 6(f)) which shows an absorbance at 254 nm. This compound may overlap with the original DLC-II and be responsible for the UV absorbance peak (Figure 3).

The $^1H$ NMR performed on DLC-II supports the proposed structure showing the vinylic hydrogens, the methoxy methylene, and the methyl protons.

(2) The DLC compound represented by peak III was subjected to EI mass spectrometry as described above. The mass spectrum pattern displayed several large peaks at 45, 87, 89, 133, 227 a.u. and several smaller peaks at 273, 315 and 360 a.u. (Figure 7). High resolution measurements of the peaks 133 and 227 lead to the accurate masses of 133.086 and 227.201 respectively. These masses correspond to the composition of $C_6H_{13}0_3$ and $C_{14}H_{27}0_2$ respectively. Treatment of the compound with $D_20$ did not change the resulted spectrum, indicating the absence of any OH or NH groups in the molecule. The presence of a small peak at the 360 a.u., corresponding to the sum of 133 and 227, suggested that this might be the molecular ion of this DLC (Figure 8). Further analysis of the mass spectral data leads to several tentative structures, which, by fragmentation in the mass spectrometer, can result in the fragments represented by the above peaks (Figure 8). The structures which can be considered for DLC-III are shown in Figure 9. All these structures are methylether derivatives of hydroxy fatty acids. Organic synthesis of the suggested compounds and comparison of their spectroscopic behaviour and biological activity with the present results would ascertain the exact structure.

EXAMPLE 3

**Activity of DLC-II and DLC-III**

As described in the BACKGROUND OF THE INVENTION the digitalis group compounds inhibit Na,K-ATPase activity. As may be seen in Figure 10, both DLC-II and DLC-III inhibit [$^3H$]-ouabain binding in a dose dependent mode. Inhibition of Na,K-ATPase activity is summarized in TABLE 1, given in arbitrary units.

TABLE 1

| | Na+, K+-ATPase activity (% of control) | |
|---|---|---|
| Control | | 100 |
| DLC-I | 1 | 91 |
| | 5 | 88 |
| | 50 | 86 |
| DLC-II | 1 | 90 |
| | 2 | 67 |
| | 3 | 71 |
| | 5 | 51 |
| | 8 | 10 |
| DLC-III | 2 | 58 |
| | 3 | 41 |
| | 5 | 15 |

Since both DLC-II (compounds of formula I) and DLC-III (compounds of formula II) inhibit the Na,K-ATPase activity and [$^3$H]-ouabain binding, they are considered digitalis-like compounds. Thus, the present endogenous compounds appear to share the biological activities attributed to known cardiac glycosides, as described above.

## Claims

1. A compound of the formula:

$$R_1\text{-}CH=CH\text{-}\underset{OH}{CH}\text{-}CH_2\text{-}\underset{OH}{CH}\text{-}(CH_2)_n\text{-}COOH \quad (I)$$

wherein $R_1$ represents a $C_{1-6}$ straight or branched alkyl radical and n is an integer of from 2 to 11, and pharmaceutically acceptable salts thereof.

2. A compound according to claim 1 wherein $R_1$ represents a $C_{3-6}$ straight or branched chain alkyl.

3. A compound according to claim 2 wherein $R_1$ is propyl.

4. Compounds according to any one of claims 1 to 3 wherein n is 9.

5. 11,13-Dihydroxy-14-octadecaenoic acid and pharmaceutically acceptable salts thereof.

6. A compound of the formula:

$$\begin{array}{l} R \\ | \\ CH\text{-}OCH_3 \\ | \\ CH\text{-}OCH_3 \\ | \\ CH_2\text{-}OCH_3 \end{array} \qquad (II)$$

wherein R represents an alkylenecarboxy, alkylenecarbonyloxy-alkyl, carbonyloxy-alkyl or alkyleneoxyalkylene-carbonyloxyalkyl radical.

7. A compound according to claim 6 wherein R is the radical $-CH_2-(-CH_2-)_{12}-COOH$ or pharmaceutically acceptable salts thereof.

8. A compound according to claim 6 wherein R is the radical $-CH_2-OCO(CH_2)_{11}CH_3$.

9. A compound according to claim 6 wherein R is the radical $-OCO(CH_2)_{12}CH_3$.

10. A compound according to claim 6 wherein R is the radical radical

$$-CH_2-O-(CH_2)_{10}-\overset{O}{\overset{\|}{C}}-OC_2H_5.$$

11. A pharmaceutical composition comprising as active ingredient a compound according to any one of claims 1 to 5, or a pharmaceutically acceptable salt thereof in a pharmaceutically acceptable carrier.

12. A pharmaceutical composition comprising as active ingredient at least one compound according to any one of claims 6 to 10 or a pharmaceutically acceptable salt thereof, in a pharmaceutically acceptable carrier.

13. A pharmaceutical composition comprising as active ingredient a mixture of at least one compound according to any one of claims 1 to 5 or a pharmaceutically acceptable salt thereof, with at least one compound according to any one of claims 6 to 10 in a pharmaceutically acceptable carrier.

14. A pharmaceutical composition according to any one of claims 11 to 13 suitable for oral or parenteral administration.

15. A pharmaceutical composition according to claim 14 in unit dosage form.

16. A compound according to any one of claims 1 to 10 for use in therapy for treating or preventing cardiac malfunction or renal malfunction.

Claims for the following Contracting States: GR, ES

1. Process for isolating a product having digitalis-like activity which comprises subjecting dried mammalian plasma to extraction with methanol, washing the methanolic extract with a volatile liquid aliphatic hydrocarbon, evaporating the washed methanolic extract to dryness, subjecting the dried extract to chromatographic separation, and isolating fractions showing digitalis-like activity as determined by bioassay.

2. Process according to claim 1 wherein the product isolated comprises a compound of the formula:

$$R_1-CH=CH-\underset{OH}{\underset{|}{CH}}-CH_2-\underset{OH}{\underset{|}{CH}}-(CH_2)_n-COOH \quad (I)$$

wherein R represents a $C_{1-6}$ straight or branched alkyl radical and n is an integer of from 2 to 11, or a pharmaceutically acceptable salt thereof.

3. A process according to claim 2 wherein $R_1$ represents a $C_{3-6}$ straight or branched chain alkyl.

4. A process according to claim 3 whererin $R_1$ is propyl.

5. a process according to any one of claims 2 to 4 wherein n is 9.

6. A process according to claim 1 wherein the product isolated comprises 11, 13-dihydroxy-14-octadecaenoic acid or a pharmaceutically acceptable salt thereof.

7. A process according to claim 1 where the product isolated comprises a compound of the formula:

$$\begin{array}{l} R \\ | \\ CH-OCH_3 \\ | \\ CH-OCH_3 \\ | \\ CH_2-OCH_3 \end{array} \qquad (II)$$

- 16 -

wherein R represents an alkylenecarboxy, alkylenecarbonyloxy-alkyl, carbonyloxy-alkyl or alkyleneoxyalkylene-carbonyloxyalkyl radical.

8. A process according to claim 7 wherein R is the radical $-CH_2-(-CH_2-)_{12}-COOH$, or a pharmaceutically acceptable salt thereof.

9. A process according to claim 7 wherein R is the radical $-CH_2-OCO(CH_2)_{11}CH_3$, $-OCO(CH_2)_{12}CH_3$, or

$$-CH_2-O-(CH_2)_{10}-\overset{O}{\overset{\|}{C}}-OC_2H_5.$$

10. A process for producing a pharmaceutical composition comprising, as active ingredient, one or more products produced by a process according to any one of claims 1 to 9, or a pharmaceutically acceptable salt thereof, which comprises mixing the said product with a pharmaceutically acceptable carrier.

11. A process according to claim 10 in which the said active ingredient used comprises a mixture of at least one compound as defined in any one of claims 2 to 6 or a pharmaceutically acceptable salt thereof, with at least one compound as defined in any one of claims 7 to 9.

12. A process according to claims 10 or 11 in which the carrier used is suitable for oral or parenteral administration.

13. A process according to claim 12 in which the composition is made in unit dosage form.

YISSUM

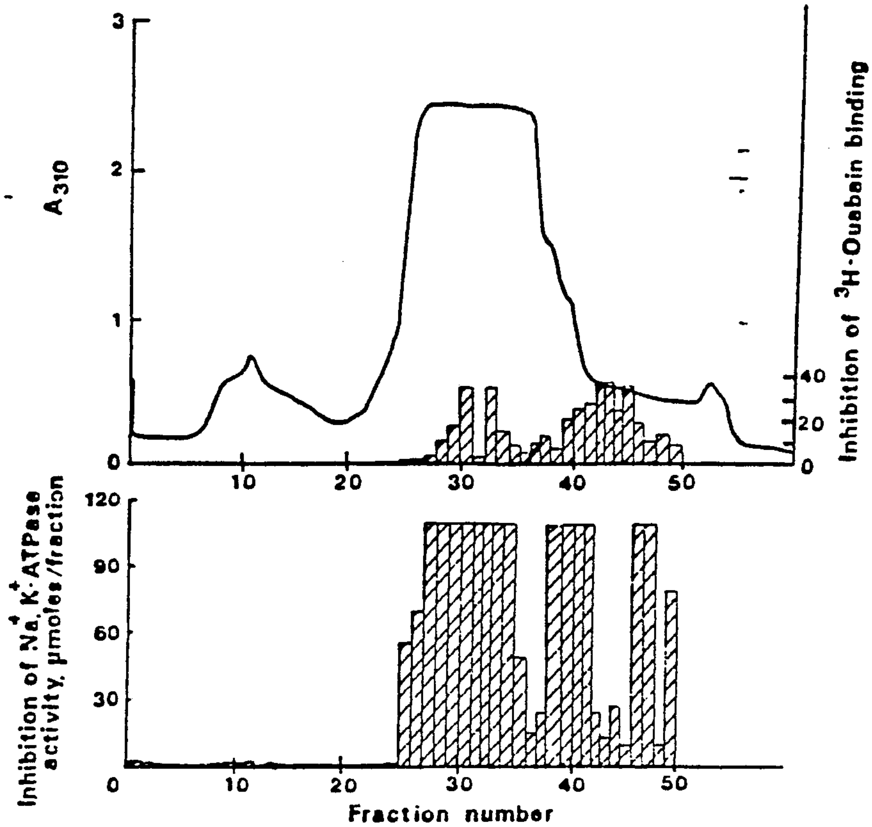


Figure 1

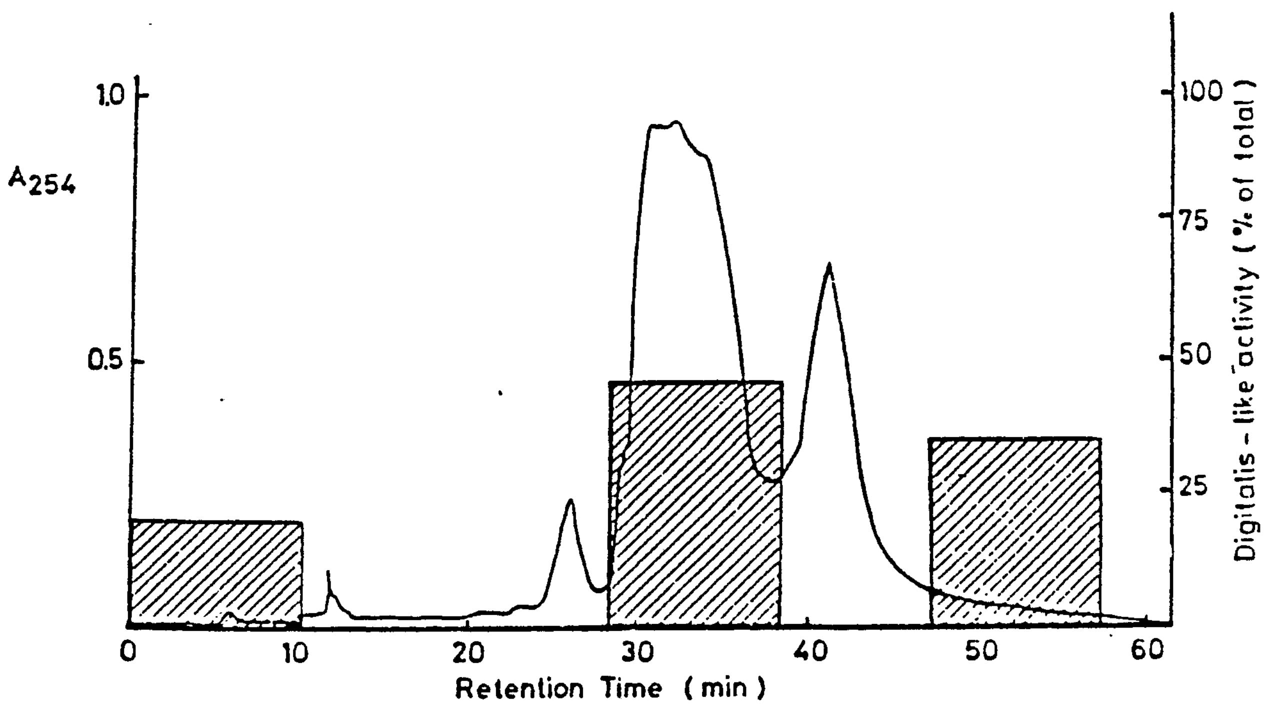


Figure 2

YISSUM

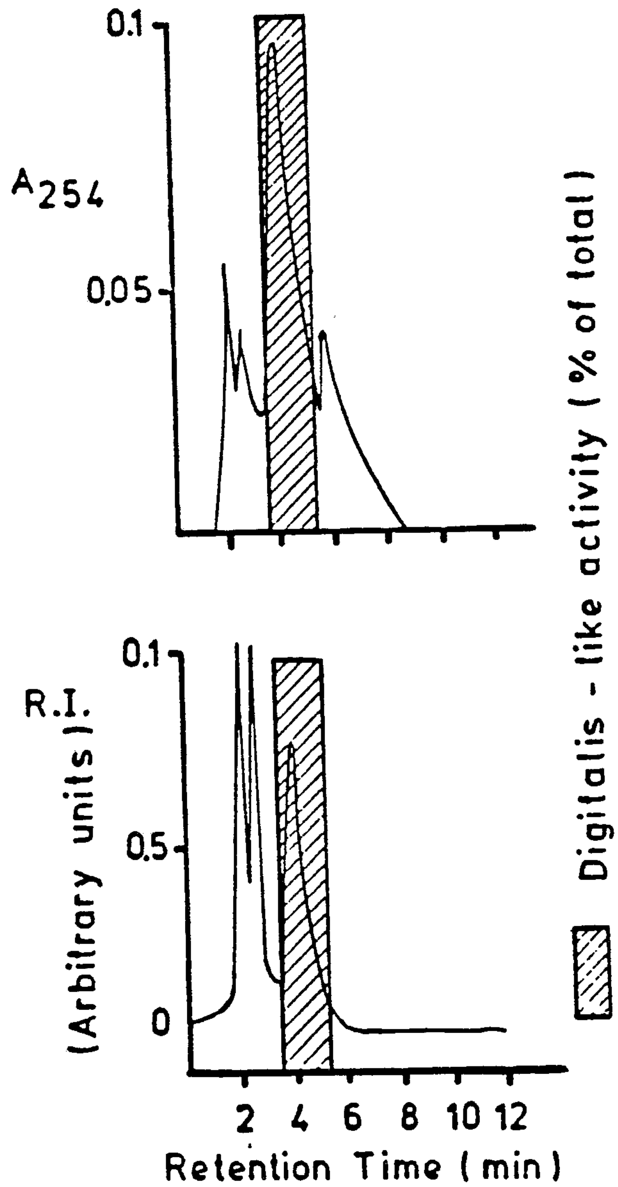


Figure 3

YISSUM

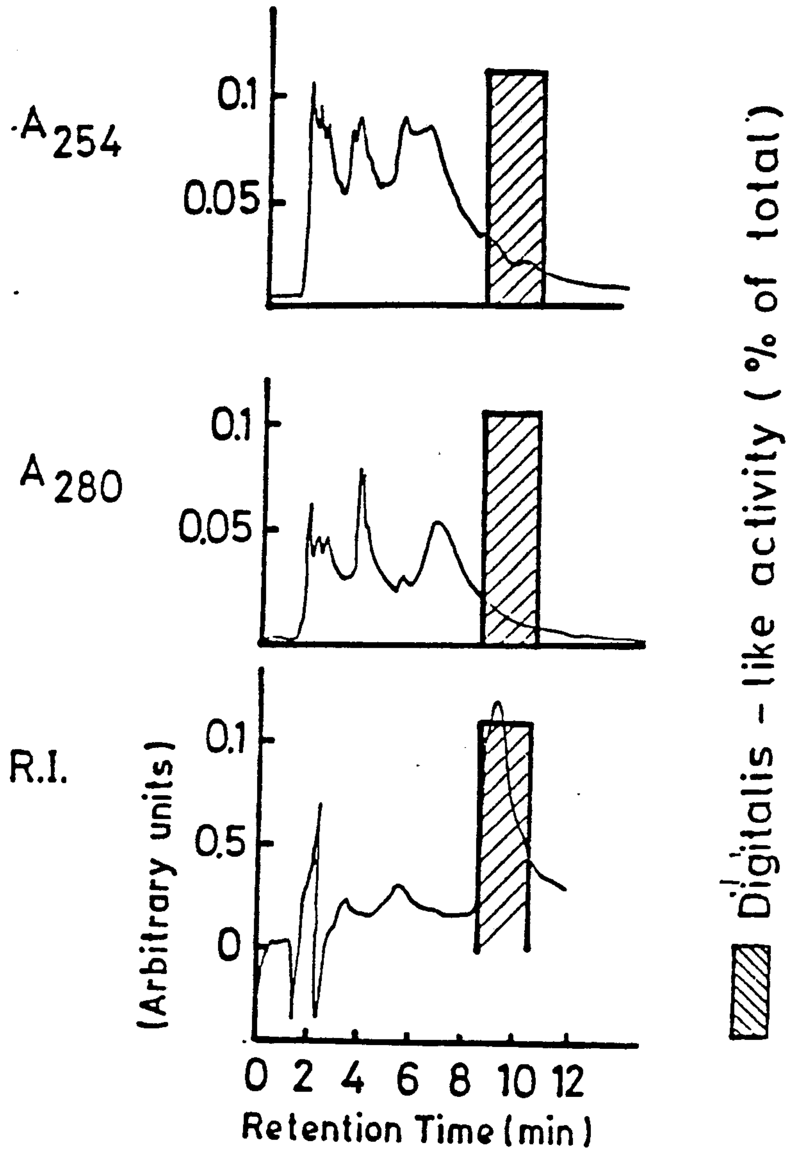


Figure 4

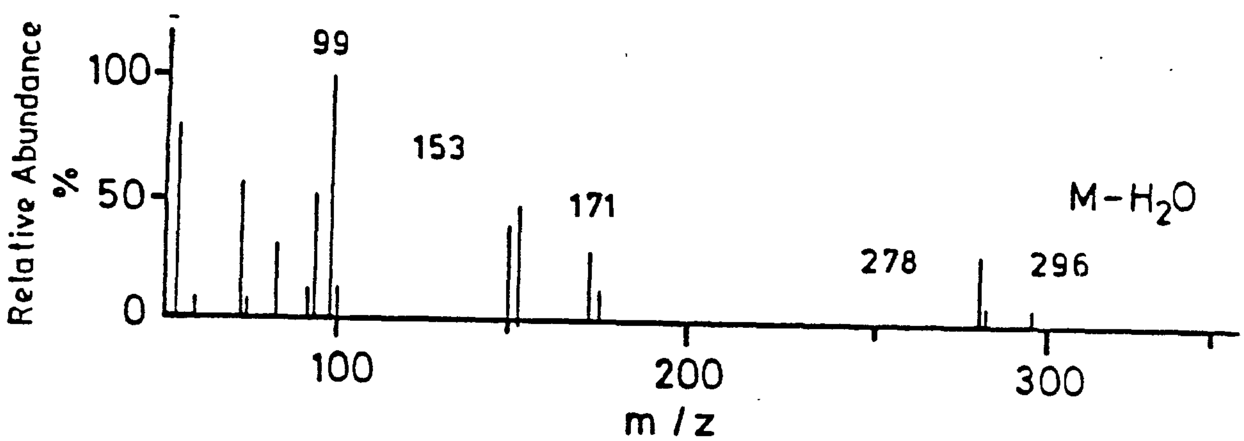
Relative Abundance %
100
50
0
99
153
171
278
296
M-$H_2O$
100
200
300
m / z

Figure 5

YISSUM

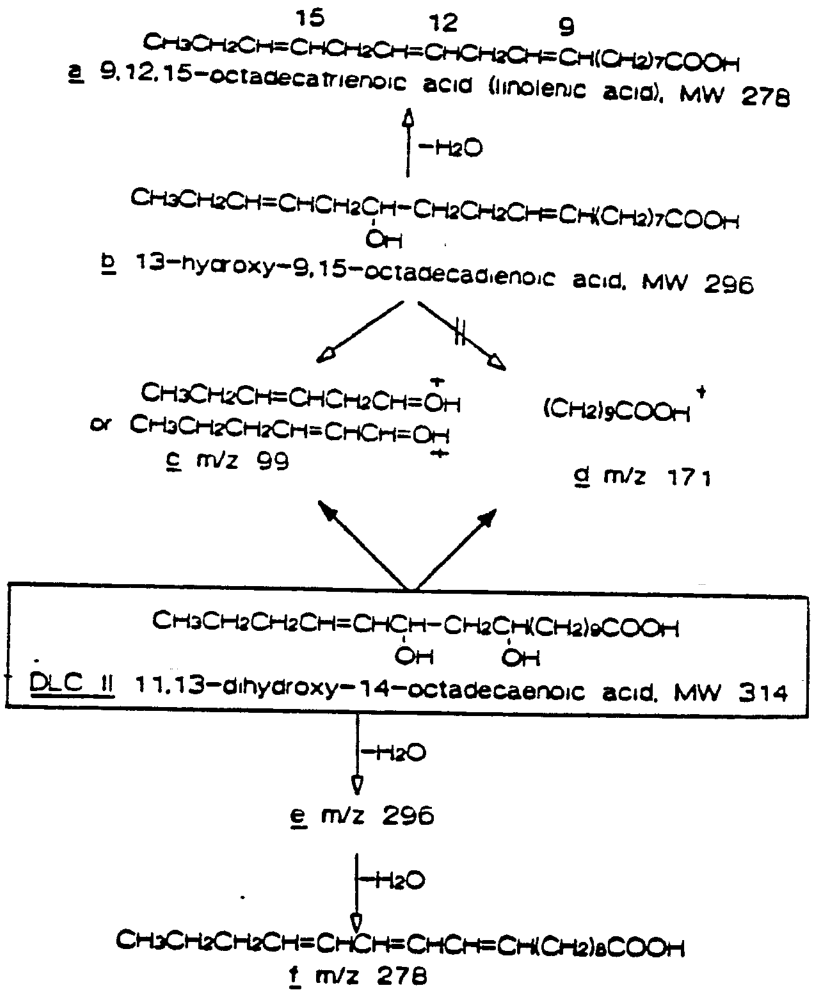


a 9,12,15-octadecatrienoic acid (linolenic acid), MW 278

b 13-hydroxy-9,15-octadecadienoic acid, MW 296

or

c m/z 99

d m/z 171

DLC II 11,13-dihydroxy-14-octadecaenoic acid, MW 314

e m/z 296

f m/z 278

Figure 6

YISSUM

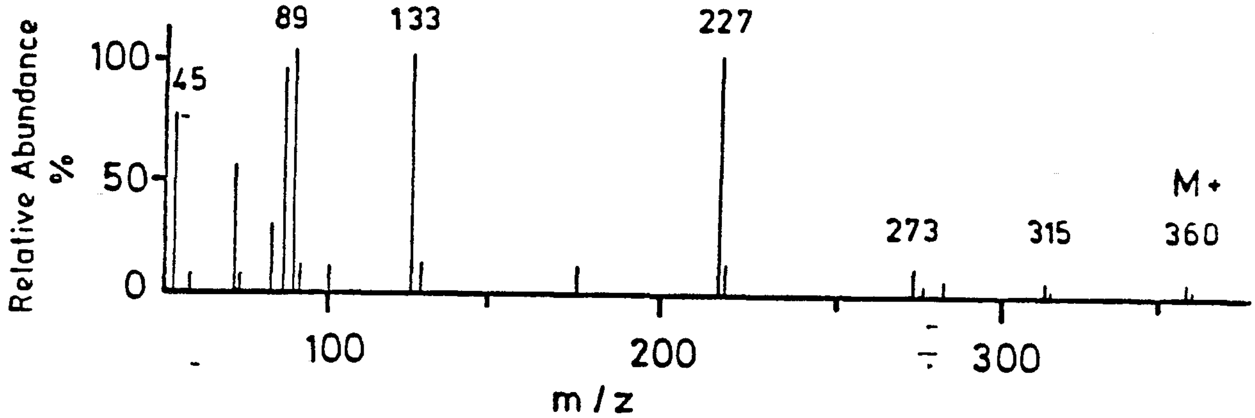


Figure 7

YISSUM

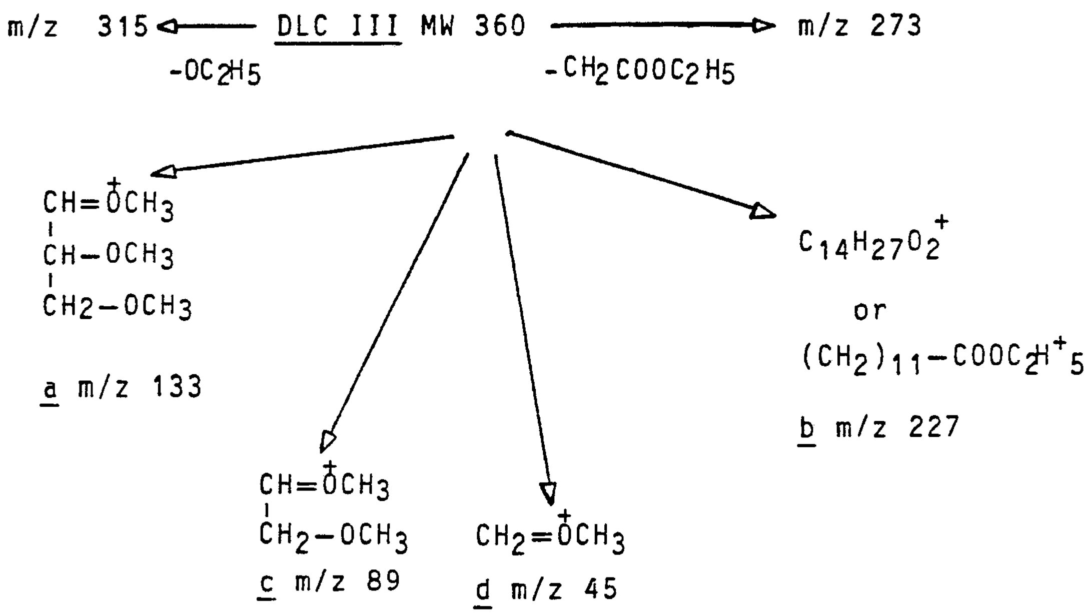


Figure 8

YISSUM

$CH_2(CH_2)_{12}COOH$
$CH–OCH_3$
$CH–OCH_3$
$CH_2–OCH_3$

DLC IIIa

$O$
$(CH_2)_{11}–C–O–C_2H_5$
$CH–OCH_3$
$CH–OCH_3$
$CH_2–OCH_3$

DLC IIId

$CH_2–OCO(CH_2)_{11}CH_3$
$CH–OCH_3$
$CH–OCH_3$
$CH_2–OCH_2$

DLC IIIb

$OCO(CH_2)_{12}CH_3$
$CH–OCH_3$
$CH–OCH_3$
$CH_2–OCH_3$

DLC IIIc

Figure 9

YISSUM

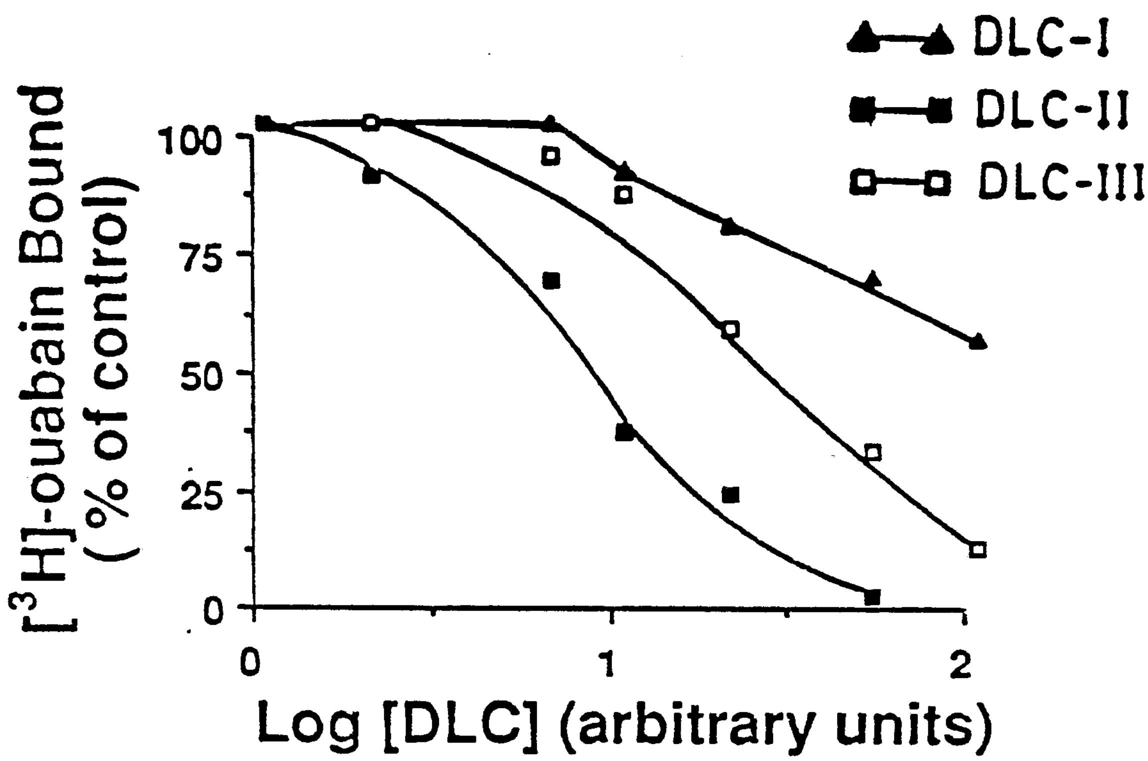


Figure 10

European Patent Office

# EUROPEAN SEARCH REPORT

Application number

EP 90301136.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 86, no. 17, April 25, 1977, Columbus, Ohio, USA GAKHOKIDZE, R.A. et al. "Isomerization of dimethylxylose to dimethyl-o-xylosaccharinic acid" page 576, abstract-no. 121 646z & Ref.Zh., Khim 1976; 10th Collective, Index, Formulas, page 4089F, column 3, lines 111-113 -- | 6 | C 07 C 59/42<br>C 07 C 59/125<br>C 07 C 69/708<br>A 61 K 31/19<br>A 61 K 31/22 |
| A | CHEMICAL ABSTRACTS, vol. 108, no. 11, March 14, 1988, Columbus, Ohio, USA TAMURA, MASAAKI et al. "Specific endogenous sodium-potassium ATPase inhibitor purified from bovine adrenal" page 308, abstract-no. 90 759e & Biochem.Biophys.Res.Commun. 1987, 149(2), 468-74 -- | 11-16 | TECHNICAL FIELDS SEARCHED (Int Cl.5)<br>C 07 C 59/00<br>C 07 C 69/00 |
| D,A | CHEMICAL ABSTRACTS, vol. 103, no. 19, November 11, 1985, Columbus, Ohio, USA KELLY,RALPH A. et al. "Characterization of digitalis-like factors in human plasma. Interactions with sodium-potassium ATPase and cross-reactivity with cardiac glycoside-specific antibodies." page 486, abstract-no. 158 126a & J.Biol.Chem. 1985, 260(21) | 11-16 | |

The present search report has been drawn up for all claims

| Place of search | Date of completion of the search | Examiner |
|---|---|---|
| VIENNA | 12-04-1990 | HOFBAUER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application number

-2-

EP 90301136.9

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.5) |
| | 11396-405<br>-- | | |
| A | CHEMICAL ABSTRACTS, vol. 93, no.7, August 18, 1980, Columbus, Ohio, USA<br>HUSAIN, SHAHID et al. "New hydroxy fatty acid from seed oil of Baliospermum axillare" page 502, abstract-no. 66 020h<br>& Phytochemistry 1980, 19(1), 75-7<br>-- | 1 | |
| A | CHEMICALS ABSTRACTS, vol. 85, no. 25, December 20, 1976, Columbus, Ohio, USA<br>GAKHOKIDZE, R.A. "Acidic reaction of hydroxy aldehydes, hydroxy ketones, and aldoses according to the Danilov and Venus-Danilova reaction" page 581, abstract-no. 192 996d<br>& Zh.Obshch.Khim. 1976, 46(7) 1620-8<br>---- | 6 | TECHNICAL FIELDS SEARCHED (Int Cl.5) |

The present search report has been drawn up for all claims

| Place of search | Date of completion of the search | Examiner |
|---|---|---|
| VIENNA | 12-04-1990 | HOFBAUER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document